# EUROPÄISCHE PATENTANMELDUNG

(11) **EP 1 216 687 A2**
(43) Veröffentlichungstag der Anmeldung: **26.06.2002**
(21) Anmeldenummer: 01811118.7
(22) Anmeldetag: 21.11.2001
(51) Int. Cl.: A61K 7/06

(54) **Haarbehandlungsmittel**

(30) Priorität: 22.12.2000 CH 252000
(71) Anmelder: Mibelle AG Cosmetics, 5033 Buchs (CH)
(72) Erfinder: Zülli, Fred, Dr., 5024 Küttingen (CH); Belser-Gisi, Esther, 5000 Aarau (CH); Muggli, Reto, 4600 Olten (CH); Karlen, Thomas, Dr., 5000 Aarau (CH)
(74) Vertreter: Rottmann, Maximilian R.

(57) **Zusammenfassung**

Es werden Haarbehandlungsmittel beschrieben, welche in einer wässerigen Phase ein Gemisch von (a) wasserlöslichen Procyanidinen und (b) öllöslichen freien Tocopherolen enthalten, wobei beide Wirkstoffe eine Affinität zum Haar aufweisen. Derartige Haarbehandlungsmittel schützen das Haar gegen Schädigungen beim Wärmetrocknen und gegen Umwelteinflüsse und/oder Haarbearbeitungen und sie schützen weitere Wirkstoffe der Haarbehandlungsmittel gegen Oxidation und/oder Degradation.

## Beschreibung

Die Erfindung betrifft ein Haarbehandlungsmittel, wie es in Anspruch 1 umschrieben ist.

Antioxidantien sind wichtige Stoffe in der Natur. Sie werden in der Kosmetik eingesetzt zum Schutze der Inhaltsstoffen in den Produkten und zum Schutz der Haut gegen oxidativen Stress.

Oxidativer Stress wird verursacht durch ROS (Reactive Oxygen Species - reaktive Sauerstoffmoleküle). ROS werden vor allem in Wasser gebildet, da das Wasser immer mit Sauerstoff gesättigt ist.

In der Kosmetik werden häufig nur öllösliche Antioxidantien eingesetzt, da eine ganze Reihe von geeigneten Stoffen zur Verfügung stehen, beispielsweise 3-tert.-Butyl-4-hydroxyanisol oder alpha-Tocopherol.

Der Einsatz wasserlöslicher Antioxidantien ist limitiert, da keine geeigneten Stoffe auf dem Markt erhältlich sind.

Vitamin C (Ascorbinsäure) ist ein wasserlösliches Antioxidans, das zwar in der Natur weit verbreitet ist, in der Kosmetik jedoch selten verwendet wird, da es sehr instabil ist.

Oligomere Procyanidine, isoliert aus Traubenkernen oder anderen Pflanzenteilen, sind sehr gute Antioxidantien, die sich zur Verwendung in Kosmetikprodukten eignen. Procyanidine sind Polyphenole auf der Basis von Catechin und Epicatechin.

In der Veröffentlichung US-A-5 648 377 ist denn auch die Verwendung von oligomeren Procyanidinen in Kombination mit Carotinoiden zur Bekämpfung von freien Radikalen in Lebensmitteln, Nahrungsergänzungsmitteln, sowie kosmetischen und pharmazeutischen Produkten vorgeschlagen.

Die Verwendung von oligomeren Procyanidinen ist jedoch nicht unproblematisch, da diese Stoffe gelöst in Wasser nur bedingt stabil sind. Daher hat sich die Verwendung dieser oligomeren Procyanidine bisher vorwiegend auf trockene Produkte, beispielsweise Kapseln oder Tabletten als Nahrungsergänzungsmittel, beschränkt.

Die Veröffentlichung EP-A1-1 086 693 beschreibt die Stabilisierung von Proanthocyanidinen in diversen Produkten, wie Tabletten, Lebensmitteln und Kosmetikprodukten mittels Vitamin B6. Bei den beschriebenen Proanthocyanidinen handelt es sich im Gegensatz zu den oligomeren Procyanidinen der vorliegenden Erfindung hauptsächlich um Dimere. Weiterhin handelt es sich bei Vitamin B6 um ein wasserlösliches Vitamin. Bei der Anwendung von Traubenkernextrakt und Vitamin B6 in kosmetischen Produkten, wie z.B. Shampoo, gemäss der genannten Veröffentlichung geht es nur um die Stabilität der Proanthocyanidine. Der Schutz von Haaren durch die Kombination von Procyanidinen mit reinem Tocopherol ist nicht ableitbar, ebenso nicht die Affinität einer solchen Kombination zum Haar. Das Beispiel eines Haarwassers mit Vitamin-E-acetat ermöglicht keinen Schutz des Haares, da Vitamin-E-acetat keine ausreichende Antioxydans-Aktivität aufweist. Vitamin-E-acetat kann jedoch in der Haut zu reinem Tocopherol gespalten werden, welches dann gewisse Aktivitäten auf weist.

In der Veröffentlichung EP-A1-0 768 079 wird der Einsatz von dimeren Proanthocyanidinen zur Förderung des Haar-Wachstums beschrieben. Das beschriebene Produkt enthält zwar zusätzlich freies Tocopherol, die Affinität des Produktes zum Haar und damit der Schutz des Haares gegen Radikale ist jedoch weder offenbart noch ableitbar und ist auch nicht Ziel der Erfindung der Veröffentlichung.

Es wurde nun überraschenderweise gefunden, dass die oligomeren Procyanidine in einer wässerigen Lösung durch Zugabe von Tocopherolen, welche in die wässerige Phase mittels kosmetischen Lösungsvermittlern, beispielsweise PEG-40 Hydrogenated Castor Oil, eingebracht werden, stabilisiert werden. Anderseits wird die Stabilität der Tocopherole durch die Gegenwart der oligomeren Procyanidine erhöht.

So bleibt beispielsweise die Antioxidans-Aktivität einer Mischung von 0,5 Gewichtsprozent oligomeren Procyanidinen und 5 Gewichtsprozent Tocopherol über 8 Monate lang stabil. Weiter zeigt diese Mischung von Antioxidantien auch eine synergistische Wirkung bei der Hemmung der UV-A-induzierten Bildung von toxischen Produkten in Squalen.

Die Schädigung von Haar durch oxidativen Stress, wie beispielsweise durch UV-Bestrahlung, ist bekannt. Kaum untersucht ist jedoch die Schädigung des Haares, welche bei seiner Wärmetrocknung, insbesondere mit einem Föhn (Haartrockner), entsteht.

Überraschenderweise wurde nun festgestellt, dass diese Beanspruchung zu schweren Schädigungen führen kann, vor allem in Anbetracht der Tatsache, dass solche Behandlungen sehr häufig durchgeführt werden.

Die Schädigung entsteht hauptsächlich dadurch, dass der gelöste Sauerstoff im Wasserfilm auf dem nassen Haar durch die Wärme aktiviert wird, d.h. es können ROS (Reactive Oxigen Spezies - reaktive Sauerstoffmoleküle) entstehen. Die ROS, beispielsweise Singulett-Sauerstoff oder Sauerstoffradikale, reagieren dann mit den Bestandteilen der Haaroberfläche und beschädigen damit die Haarstruktur. Unter anderem werden dabei die Proteine auf der Haaroberfläche oxidiert und abgebaut. Diese Proteine können dann in Form von Aminosäuren, Peptiden und Proteinen vom Haar gewaschen werden.

Eine Aufgabe der vorliegenden Erfindung war es daher, ein Haarbehandlungsmittel zu schaffen, welches das Haar gegen oxidativen Stress, insbesondere gegen Schädigungen beim Wärmetrocknen und gegen Umwelteinflüsse und/oder Haarbearbeitungen, schützt. Insbesondere sollte es auf einer wässerigen Formulierung basieren und sowohl als "Leave on"-Produkt, d.h. ein Produkt, dass auf dem Haar verbleibt, als auch als "Rinse off"-Produkt, d.h. ein Produkt, das ausgewaschen wird, wirken.

Diese Aufgabe wird durch die Merkmale des Anspruchs 1 gelöst. Bevorzugte Ausführungsformen sind in den Ansprüchen 2 bis 4 umschrieben.

In den nachstehenden Tests werden die überraschenden Eigenschaften einer Wirkstoffkombination aus oligomeren Traubenkern-Procyanidinen und Tocopherolen im einzelnen nachgewiesen. Es sind dies:
- Adsorption von Tocopherol auf der Haaroberfläche (Test 1);
- Schutz gegen Haarschädigung beim Wärmetrocknen (Test 2); und
- Schutz gegen Haarschädigung durch Meerwasser und UV-Strahlung (Test 3).

Als weitere Wirkstoffe des Haarbehandlungsmittels, welche erfindungsgemäss gegen Oxidation und/oder Degradation, insbesondere Photooxidation, geschützt werden können, sind beispielsweise zu nennen: Vitamin C und Vitamin-C-Derivate, Vitamin A und Vitamin-A-Derivate, Parfümöle, ungesättigte Lipide und Proteine.

In den nachstehenden Tests und Rezepturen sind die Produkte mehrheitlich nach der üblichen INCI-Deklaration bezeichnet. Alle Mengenangaben sind in Gewichtsprozent.

### Tests

Die folgenden Tests wurden mit kommerziell erhältlichem europäischem Haar und der folgenden Wirkstoffkombination durchgeführt:

| | |
|---|---|
| Alcohol | 30,0 % |
| Glycerin | 40,0 % |
| PEG-40 Hydrogenated Castor Oil | 10,0 % |
| Tocopherol | 5,0 % |
| Procyanidine (Grape Seed Extract) | 0,4 % |
| Wasser | ad 100 |

Für die Tests wurde diese Wirkstoffkombination mit Wasser auf 2,5 % verdünnt; sie ist im Folgenden als "Wirkstoffverdünnung" bezeichnet.

### Test 1 (Adsorption von Tocopherol auf der Haaroberfläche)

2 g Haare wurden in 20 ml der Wirkstoffverdünnung unter Rühren 10, 20 und 60 Minuten lang inkubiert. Die Haare wurden danach mit Wasser gespült und an der Luft getrocknet. Danach wurden die Haare je mit 50 ml Isopropanol extrahiert. Der Extrakt wurde dann im Vakuum eingetrocknet und anschliessend in 1 ml Ethanol gelöst. Der Gehalt an Tocopherol wurde durch eine HPLC-Analyse ermittelt.

### Resultat

| Inkubationszeit | Menge Tocopherol auf 2 g Haaren |
|---|---|
| 10 Minuten | 0,1 mg |
| 20 Minuten | 0,4 mg |
| 60 Minuten | 1,0 mg |

### Test 2 (Schutz gegen Haarschädigung beim Wärmetrocknen)

Die Haare wurden zuerst mit einem Shampoo gewaschen und gespült. Danach wurden die Haare in der Wirkstoffverdünnung während 10 Minuten inkubiert. Die Haare wurden danach mit Wasser gespült und mit einem Föhn getrocknet. Die trockenen Haare wurden dann wieder mit Wasser besprüht und erneut mit dem Föhn getrocknet. Dieser Vorgang wurde 4-mal bzw. 9-mal wiederholt. Die Haare wurden dann mit einer 2%igen wässerigen Natriumlaurylsulfat-Lösung extrahiert. Darauf wurde der Extrakt filtriert. Der Protein- und Peptidgehalt im Extrakt wurde dann nach der Methode von Bradford bestimmt. Im Kontrollversuch wurden die Haare gleich behandelt, jedoch nur in Wasser anstatt in der Wirkstoffverdünnung inkubiert.

### Resultat: Menge Protein im Extrakt

| Nach 5-maligem Föhnen | |
|---|---|
| Kontrolle | 30 µg/ml |
| Wirkstoffkombination | 7 µg/ml |

| Nach 10-maligem Föhnen | |
|---|---|
| Kontrolle | 47 µg/ml |
| Wirkstoffkombination | 19 µg/ml |

### Test 3 (Schutz gegen Haarschädigung durch Meerwasser und UV-Strahlung)

Die Haare wurden zuerst mit einem Shampoo gewaschen und gespült. Danach wurden die Haare in der Wirkstoffverdünnung während 60 Minuten inkubiert. Die Haare wurden danach mit Wasser gespült und an der Luft getrocknet. Die trockenen Haare wurden dann mit UV-Licht bestrahlt (Kontrolle: ohne UV-Strahlung). Die Haare wurden dann mit einer 2%igen wässerigen Natriumlaurylsulfat-Lösung oder mit Meerwasser extrahiert. Darauf wurde der Extrakt filtriert. Der Protein- und Peptidgehalt im Extrakt wurde dann nach der Methode von Bradford bestimmt. Im Kontrollversuch wurden die Haare gleich behandelt, jedoch nur in Wasser anstatt in der Wirkstoffverdünnung inkubiert.

### Resultat: Menge Protein in den verschiedenen Extrakten

| Natriumlaurylsulfat-Extrakt | |
|---|---|
| Ohne UV, ohne Wirkstoffkombination | 36 µg/ml |
| Ohne UV, mit Wirkstoffkombination | 10 µg/ml |
| Mit UV, ohne Wirkstoffkombination | 50 µg/ml |
| Mit UV, mit Wirkstoffkombination | 12 µg/ml |

| Meerwasser-Extrakt | |
|---|---|
| Ohne UV, ohne Wirkstoffkombination | 16 µg/ml |
| Ohne UV, mit Wirkstoffkombination | 10 µg/ml |
| Mit UV, ohne Wirkstoffkombination | 28 µg/ml |
| Mit UV, mit Wirkstoffkombination | 16 µg/ml |

### Rezepturen

| **A. Haar-Shampoo mit Traubenkern-Procyanidinen und Tocopherol** | |
|---|---|
| Sodium Laureth Sulfate 70%ig | 12,00 % |
| Cocamidopropyl Betain 35%ig | 7,00 % |
| Parfüm | 0,50 % |
| Glycerin | 0,02 % |
| Alcohol | 0,02 % |
| PEG-40 Hydrogenated Castor Oil | 0,01 % |
| Konservierungsmittel | 0,1-1,0 % |
| Tocopherol | 0,005 % |
| Procyanidine (Grape Seed Extract) | 0,0005 % |
| Wasser | ad 100 |

| **B. Haar-Conditioner mit Traubenkern-Procyanidinen und Tocopherol** | |
|---|---|
| Cetearylalkohol | 4,50 % |
| Cetrimonium Chloride | 2,50 % |
| Dimethicone Copolyol | 5,00 % |
| Parfüm | 0,50 % |
| Citronensäure | auf pH 3,5 |
| Glycerin | 2,0 % |
| Alcohol | 1,0 % |
| PEG-40 Hydrogenated Castor Oil | 0,5 % |
| Tocopherol | 0,25 % |
| Procyanidine (Grape Seed Extract) | 0,10 % |
| Konservierungsmittel | 0,1-1,0 % |
| Wasser | ad 100 |

| **C. Haarspitzen-Fluid mit Traubenkern-Procyanidinen und Tocopherol** | |
|---|---|
| Dimethicone | 25,00 % |
| Cyclomethicone | 71,00 % |
| Dimethicone Copolyol | 2,00 % |
| Parfüm | 1,00 % |
| Glycerin | 0,2 % |
| Alcohol | 0,4 % |
| PEG-40 Hydrogenated Castor Oil | 0,4 % |
| Tocopherol | 0,05 % |
| Konservierungsmittel | 0,1-1,0 % |
| Procyanidine (Grape Seed Extract) | 0,05 % |

| **D. Haarspray-Aerosol mit Traubenkern-Procyanidinen und Tocopherol** | |
|---|---|
| Acrylates Copolymer | 3,00 % |
| 2-Aminomethyl-propanol | 0,70 % |
| Wasser | 20,00 % |
| Glycerin | 0,02 % |
| PEG-40 Hydrogenated Castor Oil | 0,02 % |
| Tocopherol | 0,001 % |
| Procyanidine (Grape Seed Extract) | 0,0005 % |
| Dimethylether | 40,00 % |
| Wasser | ad 100 |

| **E. Styling-Gel mit Traubenkern-Procyanidinen und Tocopherol** | |
|---|---|
| Carbomer | 1,00 % |
| Natronlauge 30%ig | 1,10 % |
| PVP/VA-Copolymer | 4,00 % |
| Glycerin | 5,00 % |
| Alcohol | 2,0 % |
| PEG-40 Hydrogenated Castor Oil | 1,0 % |
| Tocopherol | 0, 5 % |
| Procyanidine (Grape Seed Extract) | 0,1 % |
| Konservierungsmittel | 0,1-1,0 % |
| Wasser | ad 100 |

| **F. Conditioner mit Traubenkern-Procyanidinen, Tocopherol und Retinyl Palmitat** | |
|---|---|
| Cetearylalkohol | 4,50 % |
| Cetrimonium Chloride | 2,50 % |
| Dimethicone Copolyol | 5,00 % |
| Parfüm | 0,50 % |
| Citronensäure | auf pH 3,5 |
| Ceteareth-20 | 1,00 % |
| Glycerin | 2,0 % |
| Alcohol | 2,0 % |
| PEG-40 Hydrogenated Castor Oil | 1,0 % |
| Tocopherol | 0,001 % |
| Procyanidine (Grape Seed Extract) | 0,5 % |
| Retinyl Palmitate | 1,00 % |
| Konservierungsmittel | 0,1-1,0 % |
| Wasser | ad 100 |

| **G. Haartonikum mit Traubenkern-Procyanidinen, Tocopherol und Borage-ÖI** | |
|---|---|
| Alcohol | 30,00 % |
| Borage Seed Oil | 2,5 % |
| PEG-60 Hydrogenated Castor Oil | 1,00 % |
| PEG-40 Hydrogenated Castor Oil | 1,00 % |
| Parfüm | 0,50 % |
| Tocopherol | 0,25 % |
| Procyanidine (Grape Seed Extract) | 0,25 % |
| Konservierungsmittel | 0,1-1,0 % |
| Wasser | ad 100 |

| **H. Haarwasser zur Behandlung der Kopfhaut mit Traubenkern-Procyanidinen und Tocopherol** | |
|---|---|
| Konservierungsmittel | 0,1...1,0 % |
| Parfüm | 0,5 % |
| Polysorbate-20 | 2,0 % |
| Glycerin | 3,0 % |
| Sorbitol | 8,0 % |
| Alcohol | 2,0 % |
| PEG-40 Hydrogenated Castor Oil | 2,00 % |
| Tocopherol | 1,0 % |
| Procyanidine (Grape Seed Extract) | 0,1 % |
| Konservierungsmittel | 0,1-1,0 % |
| Wasser | ad 100 |

| **I. Non-aerosol-Haarspray mit Traubenkern-Procyanidinen und Tocopherol** | |
|---|---|
| Acrylates Copolymer | 3,00 % |
| 2-Aminomethyl-propanol | 0,70 % |
| Alcohol | 10,00 % |
| Glycerin | 0,6 % |
| PEG-40 Hydrogenated Castor Oil | 0,3 % |
| Tocopherol | 0,1 % |
| Procyanidine (Grape Seed Extract) | 0,2 % |
| Konservierungsmittel | 0,1-1,0 % |
| Wasser | ad 100 |

## Patentansprüche

1. Haarbehandlungsmittel zum Schutz gegen Schädigungen des Haares beim Wärmetrocknen und gegen Umwelteinflüsse und/oder Haarbearbeitungen sowie zum Schutz weiterer Wirkstoffe des Haarbehandlungsmittels gegen Oxidation und/oder Degradation, **dadurch gekennzeichnet, dass** es in einer wässerigen Phase ein Gemisch von (a) wasserlöslichen Procyanidinen und (b) öllöslichen freien Tocopherolen enthält, wobei beide Wirkstoffe eine Affinität zum Haar aufweisen.

2. Haarbehandlungsmittel nach Anspruch 1, **dadurch gekennzeichnet, dass** es einen Gehalt von 0,00005 bis 5 Gewichtsprozent an wasserlöslichen Procyanidinen (a) und von 0,00025 bis 10 Gewichtsprozent an öllöslichen freien Tocopherolen (b) aufweist.

3. Haarbehandlungsmittel nach Anspruch 1 oder 2, **dadurch gekennzeichnet, dass** die wasserlöslichen Procyanidine (a) in oligomerer Form vorliegen.

4. Haarbehandlungsmittel nach Anspruch 3, **dadurch gekennzeichnet, dass** die wasserlöslichen Procyanidine (a) 5 bis 50 Untereinheiten umfassen.
